# EUROPEAN PATENT SPECIFICATION

(11) **EP 2 459 049 B1**
(45) Date of publication and mention of the grant of the patent: **28.08.2019**
(21) Application number: 10805081.6
(22) Date of filing: 29.07.2010
(51) Int. Cl.: A61B 1/005, A61B 17/34

(54) **DEFLECTABLE INSTRUMENT PORTS**
ABLENKBARE INSTRUMENTANSCHLÜSSE
PORTS POUR INSTRUMENT DÉVIABLES

(30) Priority: 29.07.2009 US 229275 P; 22.02.2010 US 306946 P
(43) Date of publication of application: 06.06.2012
(73) Proprietor: TransEnterix Surgical, Inc., Morrisville, NC 27560 (US)
(72) Inventor: PAGE, Brett, Morrisville, NC 27560 (US); CASTRO, Salvatore, Morrisville, NC 27560 (US)
(74) Representative: Millburn, Julie Elizabeth
(86) International application number: PCT/US2010/043799
(87) International publication number: WO 2011/014711

(56) References cited:
- US-A1- 2003 236 505
- US-A1- 2005 004 515
- US-A1- 2005 228 224
- US-A1- 2005 273 133
- US-A1- 2006 025 652
- US-A1- 2006 025 652
- US-A1- 2007 021 737
- US-A1- 2007 299 387
- US-A1- 2008 046 000
- US-A1- 2008 188 868
- US-A1- 2008 255 519
- US-B1- 6 458 077
- US-B1- 6 478 028

## Description

### TECHNICAL FIELD OF THE INVENTION

The present invention relates to the field of access devices and ports through which flexible medical instruments may be introduced into a body cavity and steered or deflected.

### BACKGROUND

Surgery in the abdominal cavity is frequently performed using open laparoscopic procedures, in which multiple small incisions, trocar punctures, or ports are formed through the skin and underlying muscle and peritoneal tissue to gain access to the peritoneal site using the various instruments and scopes needed to complete the procedure. The peritoneal cavity is typically inflated using insufflation gas to expand the cavity, thus improving visualization and working space. Further developments have led to systems allowing such procedures to be performed using only a single port.

In single port surgery ("SPS") procedures, it is useful to position a device within the incision to give sealed access to the operative space without loss of insufflation pressure. Ideally, such a device provides sealed access for multiple instruments while avoiding conflict between instruments during their simultaneous use. Some multi-instrument access devices suitable for use in SPS procedures and other laparoscopic procedures are described in co-pending U.S. Application No. 11/804,063 [US 2007/0299387] ('063 application), filed May 17, 2007, entitled SYSTEM AND METHOD FOR MULTI-INSTRUMENT SURGICAL ACCESS USING A SINGLE ACCESS PORT, U.S. Application No. 12/209,408 [US 2009/0227843], filed September 12, 2008, entitled MULTI-INSTRUMENT ACCESS DEVICES AND SYSTEMS, U.S. Application No. 12/511,043 [US 2011/0060183], filed July 28, 2009, entitled MULTI-INSTRUMENT ACCESS DEVICES AND SYSTEMS, and U.S. Application No. 12/649,307 [US 2011/0230723], filed December 29, 2009, entitled ACTIVE INSTRUMENT PORT SYSTEM FOR MINIMALLY-INVASIVE SURGICAL PROCEDURES. The aforementioned patent applications describe access devices or systems that incorporating instrument delivery tubes having deflectable distal ends. Flexible instruments passed through the instrument delivery tubes are steered by actively deflecting the deflectable instrument delivery tubes. The present application describes instrument delivery tubes that may be used for this purpose, or that may be used with other single- or multi-instrument trocars, access ports, or intravascular access systems including those known to those skilled in the art.

US 2005/0004515 A1 discloses a surgical access device 100 comprising an access sheath 102 including an elongated body 105 and a steerable region or portion 106. US 2005/0004515 A1 discloses an actuator or actuation hand piece 500 having at least one thumbwheel 508 that allows a user to deflect the steerable portion 106 of the access sheath 102. The steerable portion 106 may be deflected through the action of a tensioning device 116, such as a pull wire or control wire. As the thumbwheels 508a and 508b are rotated, the tensioning device 116 imparts a pulling force on the steerable portion 106 of sheath 102, thereby causing portion 106 to deflect. US 2005/0004515 A1 discloses that the access sheath may comprise a plurality of pull wires attached to a plurality of thumbwheels of an actuation hand-piece to deflect the steerable portion of the sheath in different directions.

### BRIEF DESCRIPTION OF THE DRAWINGS

Fig. 1 is a perspective view showing two exemplary ports not according to the invention;
Fig. 2 is a plan view of the port shown in Fig. 1;
Fig. 3 is a plan view similar to Fig. 2 showing an alternate port not according to the invention;
Fig. 4 is a longitudinal cross-section view of a proximal portion of an instrument delivery tube, an actuator, and a distal portion of a control tube;

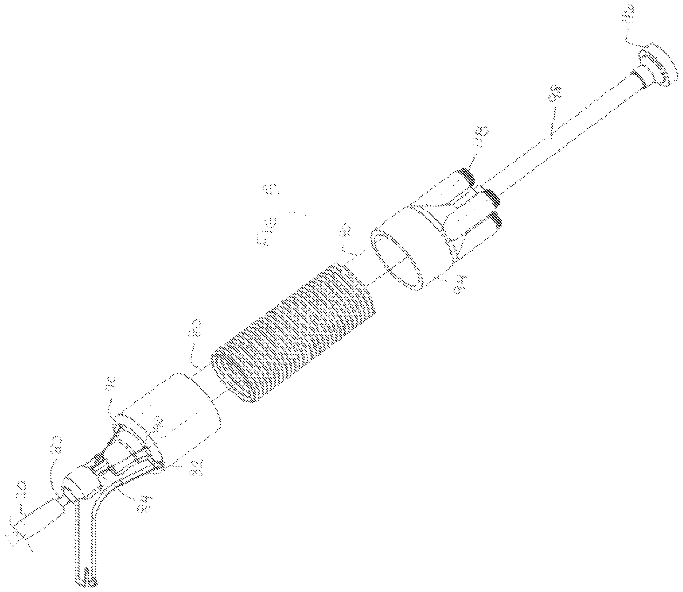

Fig. 5 is an exploded view of the actuator of Fig. 4;
Fig. 6A is a perspective view showing instruments in use in a multi-access system utilizing the port of Fig. 1;
Fig. 6B is similar to Fig. 6A and shows deflection of an instrument used in one of the ports;
Fig. 7A is a perspective view alternative embodiment of an active, flexible, port, not according to the invention showing two of the ports positioned side by side;
Fig. 7B is a perspective view of modified version of the Fig. 7A port;
Fig. 7C is a perspective view showing a distal tip of the port of Fig. 7B, illustrating one arrangement for securing the distal ends of the actuation elements. The distal tip is shown as transparent to allow the components beneath it to be seen.
Fig. 8 is an exploded view of the actuator of the Fig. 7A port;
Fig. 9 is a perspective view of the distal end of the Fig. 7A port;
Fig. 10 is an exploded view of the handle rotation mechanism of the port of Fig. 7A;
Fig. 11 is an exploded view of the handle and coupler of Fig. 10;
Fig. 12 is a cross-section view of the coupler and housing of Fig. 10;
Fig. 13 is yet another embodiment of an active, flexible port not according to the invention;
Fig. 14 is a perspective view of the distal end of the port of Fig. 13, showing one segment separated from the rigidizable section;
Fig. 15 is a perspective view of the handle of the Fig. 13 port;
Fig. 16 is a perspective view of an alternative port according to the invention having an articulation joint;
Fig. 17A is a perspective view of the distal end of the port of Fig. 14;
Fig. 17B is a side elevation view of the articulation joint of Fig. 16;
Fig. 18 is a cross-section view of the proximal section of the rigid tube of the Fig. 16 embodiment;
Fig. 19 is a perspective view showing the actuator for the articulation joint of Fig. 16;
Fig. 20A is an elevation view of the port of Fig. 16 in the straight position;
Fig. 20B is similar to Fig. 20A but shows the port in an articulated position;
Fig. 21 is similar to Fig. 17B but shows the articulation joint in the articulated position;
Fig. 22 is a perspective view of a stabilization arm of a type that may be used to support a port of the type disclosed herein;
Fig. 23A is a perspective view showing two of the Fig 13 ports disposed through a multi-instrument access device, together with a laparoscope;
Fig. 23B is an enlarged perspective view showing the access device of Fig. 23A;
Fig. 24 is a perspective view showing two of the Fig. 16 ports disposed through a multi-instrument access device;
Fig. 25 is a perspective view showing one of the Fig. 13 ports disposed through an access device, together with a port extender and a laparoscope positioned through the port extender;

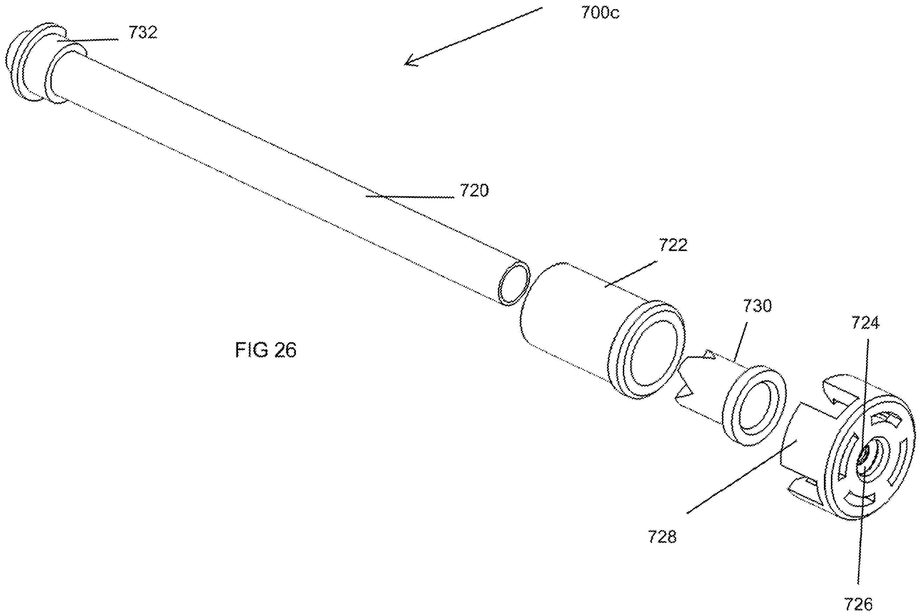

Fig. 26 is an exploded view of the port extension shown in Fig. 25.

### DETAILED DESCRIPTION

The following embodiments are instrument ports which function as deflectable, preferably sealed conduits through which flexible medical instruments are passed into the body. As will be appreciated from the discussion that follows, the ports include actuators positioned outside the body that allow active deflection of the distal ends of the ports, and thus the distal ends of the instruments passed through them. The deflectable ports described herein may extend into the body through various types of access devices suitable for use in giving access to a body cavity, including, but not limited to laparoscopic ports, trocars, cannulas, seals, multi-instrument access devices, etc., or they may extend directly through an incision.

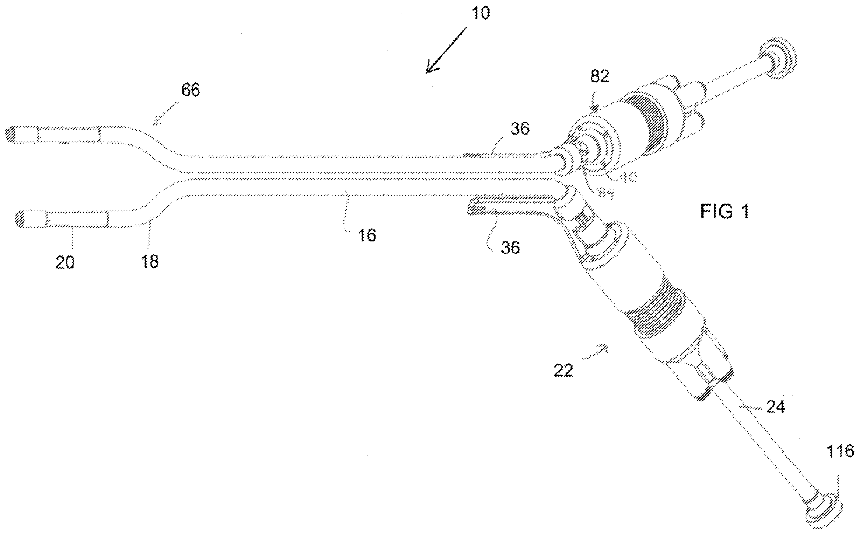

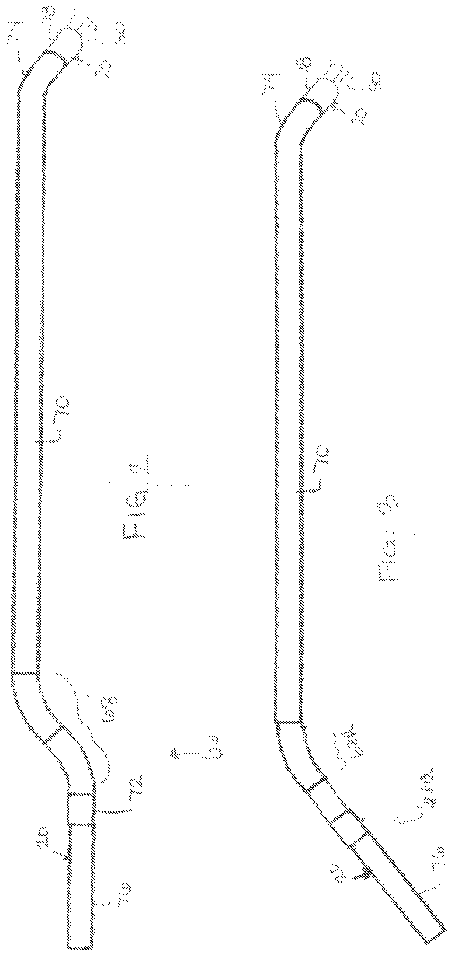

Two embodiments of deflectable instrument access ports 10 not according to the invention are shown in Fig. 1. Each such port includes an elongate instrument delivery tube 16. In the illustrated embodiment and as shown in Fig. 2, the instrument delivery tube 16 includes a flexible distal section 20. An actuator 22 on the proximal portion of the port 10 controls deflection of the flexible distal section 20 of the instrument delivery tube 16 to allow manipulation of the operative end of an instrument disposed within the instrument delivery tube 16. As will be described in detail below, the distal end of an instrument to be deployed into the body cavity via the port device 10 is inserted into a control tube 24 on the actuator 22 and then advanced into and through the instrument delivery tube 16. Manipulating the proximal handle of the instrument in turn moves the control tube 24, causing corresponding deflection of the distal end of the instrument delivery tube 16 and the instrument.

Features of the instrument delivery tube 16 will next be described with respect to Fig. 2. In the illustrated embodiment, the instrument tube 16 includes a rigid tube 18 which may be formed of stainless steel or other rigid tubing. The rigid tube 18 may be a singular tube, or a series of tubes coupled together. As shown in Fig. 2, the rigid tube 18 is manufactured to have a fixed, preformed shape that includes a generally straight main section 70 and a distal region 66 which includes a bend to create a curved or angled section 68. The curvature of the bend in the curved or angled section may be continuous or compound, and it can be formed to occupy a single plane or multiple planes.

The curved section 68 shown in Fig. 2 has an elongated S-shape, with a more proximal section that curves downwardly relative to the longitudinal axis of the main section 70 and a more distal section that curves slightly upwardly. It should be noted that the terms "downwardly", "upwardly" etc are used with reference to the drawings and not with reference to particular structures inside or outside the body cavity. The distal region 66 may additionally have a second straight section 72 distal to the curved or angled section 68. Note that while the longitudinal axis of the straight section 72 is shown parallel to that of the straight main section 70; however it may alternatively diverge towards or away from the longitudinal axis of the section 70.

For the instrument delivery tube shown in Fig. 2, the longitudinal axes of the straight shaft 70, curve 68 and distal end section 72 lie within a single plane, while a proximal bend section 74 of the tube 18 curves laterally out of that plane as well as downwardly. This arrangement helps to position control tubes 24 of adjacent instrument access devices 10 in a divergent relationship, thereby avoiding conflict between them. Various alternative shapes for the tube 18 other than those shown in the illustrated embodiments may instead be used. For example, in an alternate instrument delivery tube shown in Fig. 3, the bend may form a section 68a having a single curve or an angle extending from the straight shaft 70, rather than an s-shaped curve.

The instrument delivery tube 16 also includes a flexible inner tube 20 extending through the rigid tube 18. The inner tube 20 has distal and proximal sections 76, 78 extending beyond the distal and proximal ends, respectively, of the corresponding rigid tube 18. The inner tube20 can be made with or without a pre-formed curve or angle.

The inner tube 20 further includes a lumen for receiving an instrument that is to be used within the body. A plurality of actuation elements 80 (which in this description may also be referred to as pull wires or cables but which may take alternate forms) extend through pullwire lumens (not shown) in the wall of the inner tube 20 and are anchored near the distal end. In the preferred embodiment, each instrument delivery tube has four such wires arranged at 90 degree intervals. Other embodiments can utilize different numbers of pullwires, such as three pullwires equally spaced around each inner tube 20.

As will be discussed in detail below, the pullwires 80 are coupled to the actuator 22 (Fig. 1), which acts on the pull-wires to deflect the distal section 76 of the flexible tube 20. The flexible tube 20 is therefore constructed to be sufficiently flexible to allow the required deflection for instrument manipulation, while preferably also being resistant to kinking. In one embodiment, the flexible tube 20 is a composite tube formed using a PFTE inner liner lining its lumen, a thermal plastic sheath (having the pull wire lumens formed through it) overlaying the liner, a reinforcing layer (e.g. mesh or braid) over the thermal plastic sheath, and a second thermal plastic sheath over the reinforcing layer. In an alternate embodiment, the second thermal plastic sheath is eliminated and the reinforcing layer serves as the outer layer of the sheath. In yet another embodiment, the reinforcing layer may comprise the most inner layer of the tube. Various other embodiments, including those provided without reinforcing layers, or those having additional layers of reinforcing material or other materials can also be used.

It should be also noted that while the rigid tube 18 is beneficial for supporting the flexible tube 20 (and thus the instrument passed through it) within the body cavity, other embodiments may be provided without the rigid tube 18, and thus with only the flexible tube 20 comprising the instrument delivery tube. Such embodiments might be useful in applications where the instrument access device 10 is used with another access port having features that will support the shaft of the instrument delivery tube 16 using other elements, thus rendering the rigid tube 18 unnecessary for supporting the flexible tube 20 within the body cavity.

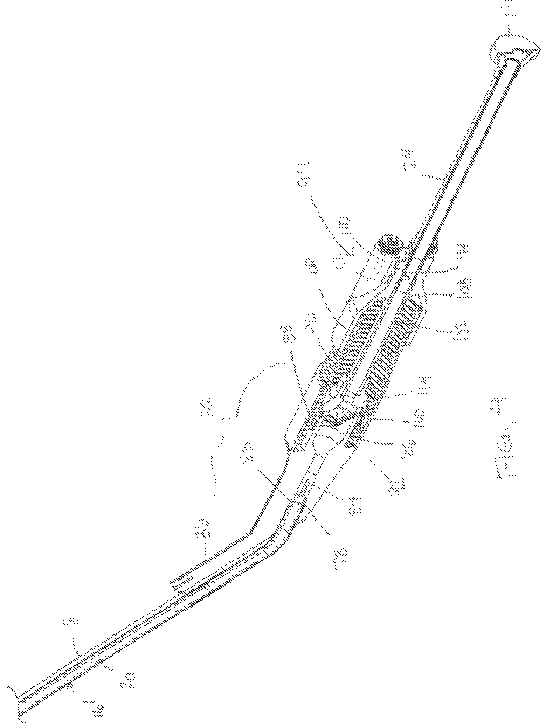

Fig. 4A shows a cross-section view of the proximal end of the instrument delivery tube 16 and actuator assembly 22. In general, the actuator assembly 22 includes a distal element 82, a proximal element 94, and a spring 96 extending between the distal and proximal elements. The rigid control tube 24 is coupled to the proximal element 94. The control tube 24 includes a lumen for receiving a medical instrument that is to be deployed through a corresponding instrument delivery tube 16. The control tube 24 may have a lubricious lining formed of PTFE or other suitable material so as to allow instruments inserted through the control tube to slide with ease.

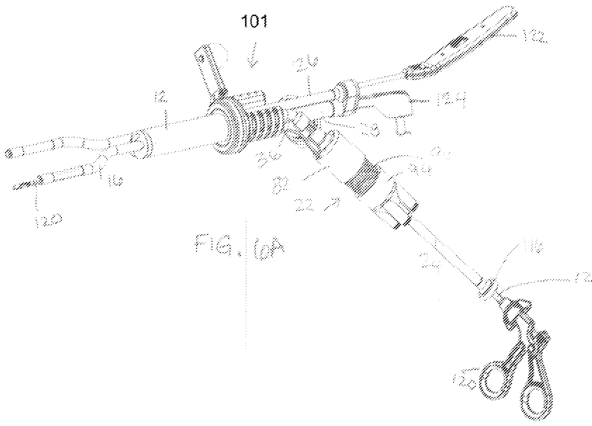

Distal element 82 is mounted to the proximal end of the rigid tube 18 of the instrument delivery tube 16. Distal element 82 may include a member 36 that allows the system 10 to be coupled to a larger access system as will be discussed in connection with Fig. 6A.

The distal element includes a lumen 83. The proximal end of the rigid tube 18 is disposed in a fixed position within the lumen 83, with the proximal end 78 of the flexible inner tube 20 extending further proximally within the lumen 83. A plurality of openings or slots 84 (one visible in Fig. 4) is formed in the distal element 82. Each slot 84 extends from the lumen 83 to the exterior of the distal element 82.

In a proximal portion of the distal element 82, the lumen 83 is surrounded by an inner cylindrical wall 86, which is itself surrounded by an outer cylindrical wall 88. The outer wall 88 defines a proximally facing cylindrical interior or receptacle, and also defines a cylindrical gap 92 between the two walls 86, 88. As best seen in Fig. 1, a plurality of through holes 90 extend from the proximal end of the gap 92 (Fig. 4) to the exterior of the proximal fitting 82. The through holes 90 and the slots 84 are radially aligned and correspond in number to the number of pullwires in the corresponding instrument delivery tube 16.

Referring again to Fig. 4, proximal element 94 includes a wall 106 defining a distally-facing cylindrical interior or receptacle 108. A lumen 110 extends from the interior 108 to the proximal face of the proximal element 94. A plurality of pullwire lumen 112 extend through the proximal element 94, preferably in parallel to the lumen 110.

The spring 96 is coupled between the proximal element 94 and the distal element 82. In the illustrated embodiment, the distal end of the spring is disposed in the proximally-facing receptacle defined by outer wall 88 of the distal element 82, and the proximal end of the spring is disposed in the distally-facing receptacle 108 of the proximal element 94.

The spring 96 is a rigid spring formed of stainless steel or other suitable materials. Components extending through the spring define a sealed instrument passage between the proximal and distal elements 94, 82. A seal, such as the cross-slit seal 100 shown in Fig. 4, is positioned in the lumen 83. This seal prevents loss of insufflation pressure through the actuator assembly 22 during times when there is not an instrument disposed in the corresponding instrument delivery tube. A length of flexible tubing, such as a Tygon tube 102, extends proximally from the seal 94. A connector 104 couples, and creates a seal between, the inner wall 86 and the tube 102.

The proximal end of the tube 102 extends into the lumen 110 of the proximal element 94. A tubular coupling 114 forms a sealed connection between the tube 102 and the control tube 24, which has a distal end disposed within the lumen 110. A seal 116 is positioned on the proximal end of the control tube 24. Seal 116 is preferably an elastomeric septum-type seal having an opening proportioned to seal against the shaft on an instrument positioned through the control tube 24.

The mechanism by which the actuator assemblies 22 control deflection of the flexible distal region of the corresponding instrument delivery tube 16 will be next be described. As discussed in connection with Fig. 2, pullwires 80 are anchored within the deflectable distal portion 76 of each flexible tube 20, and extend from the proximal portion 78 of the flexible tube 20 which, as noted in the discussion of Fig. 3, is disposed within the distal element 82 of the actuator 22. The pullwires 80 then extend from the distal element 82 and are anchored to the proximal element 94. While other arrangements can be used, in the illustrated arrangement, the pullwires 80 extend from the flexible tube 20, exit the distal element 82 via the slots 84, re-enter the distal element 82 via the through holes 90, and extend through the spring 96 into the proximal element 94. The pullwires 80 are coupled to adjustment screws 118 on the proximal element 94. The adjustment screws are rotatable to adjust the sensitivity of the actuator by increasing or decreasing the tension on the pullwires.

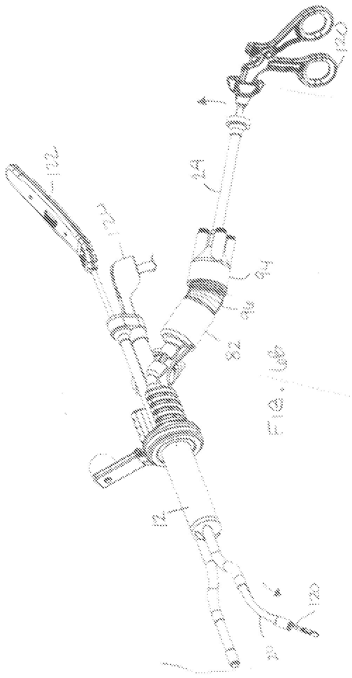

To use the port 10, an incision is formed through the skin and underlying tissue. The distal end of the instrument delivery tube 16 is inserted through the incision and into the body cavity. The actuator 22 remains outside the body. The deflectable port(s) 10 may be introduced independently or as part of a large access system which includes an access device that is seated in the incision and through which the ports 10 extend. For example, multi-instrument access systems of the type described in U.S. Application Nos. 12/209,408 [US 2009/0227843], filed September 12, 2008, and 12/511,043 [US 2011/0060183], filed July 28, 2009, may be positioned in the incision and used to provide an access point for one or more of the ports 10. In one such system 101, shown in Figs. 6A and 6B, two deflectable ports 10 are used, together with additional (in this case inactive) ports 26, 28 for receiving additional instruments. The surgeon will select instruments needed to perform a procedure within the body cavity. For example, referring again to Fig. 6A which shows a pair of deflectable ports 10, a first instrument 120 is chosen for deployment and use through a first one of the ports 10, and a second instrument (not shown) is selected for use through a second one of the ports 10. A laparoscope or endoscope 124 and an additional instrument 122 are placed in the additional ports 26, 26. In Fig. 6A, the distal ends of the scope 122 and instrument 124 are not visible, but they will extend distally from the corresponding ports of the system 101 into the body cavity.

To deploy an instrument through a deflectable instrument port 10, the distal end of the instrument I is inserted into the entry port 116 at the proximal end of the control tube 24. The instrument is advanced to pass the distal end through the actuator 22 and through the instrument delivery tube 16 until it extends from the distal end of the flexible tube 20. A seal at the entry port 116 seals against the shaft of the instrument to prevent loss of insufflations pressure. The instrument 120 may then be use for diagnosis or treatment at a treatment site in the body cavity.

When it becomes necessary for the surgeon to deflect or articulate the distal end of the instrument 120, s/he intuitively moves the handle of that instrument, causing the control tube 24 and thus the proximal element 94 to move with it. The instrument 120 may be provided with a rigid section 126 extending from the handle to optimize force transfer from the instrument 120 to the control tube 24. Movement of the control tube will cause the proximal element 94 of the actuator 22 to move relative to the distal element 82, causing the spring 96 to bend and tensioning the pullwires in accordance with the angle of the proximal element relative to the distal element. The pullwires deflect the distal portion 76 of the flexible tube 20 portion of the instrument delivery tube 16, causing corresponding deflection of the distal end of the shaft of the instrument disposed within the instrument delivery tube. Thus, to lower the distal end of the instrument as shown in Fig. 6B, the user will raise the instrument handle 120, moving the proximal portion 94 upwardly relative to the distal portion 82. This will thus apply tension to the lower pullwires, causing downward deflection of the instrument delivery tube as well as the distal end of the instrument. Lateral movement of the instrument shaft to the right will tension the corresponding side pullwire to cause the distal portion of the instrument delivery tube to bend to the left. In alternate configurations, the pullwires 80 may be routed such that the movement of the instrument tip matches the handle movement (e.g. raising the handle raises the tip, etc.). The actuator system allows combinations of vertical and lateral deflection, giving 360° deflection to the instrument delivery tube. The user may additionally advance/retract the tool 120 longitudinally within the instrument delivery tube, and/or axially rotate the instrument 120 within the instrument delivery tube when required.

Instruments suitable for use with the instrument delivery tubes include those described in co-pending U.S. Application No. 12/511,053 [US 2011/0029010], filed July 28, 2009, entitled Flexible Dissecting Forceps, and U.S. Application No. 12/511,050 [US 2011/0196418], filed July 28, 2009, entitled Flexible Medical Instruments.

It should be noted that the deflectable ports described herein may be used with any other type of access system, laparoscopic port, trocar, cannula, seal, catheter etc. suitable for use in giving access to a body cavity, or directly through an incision.

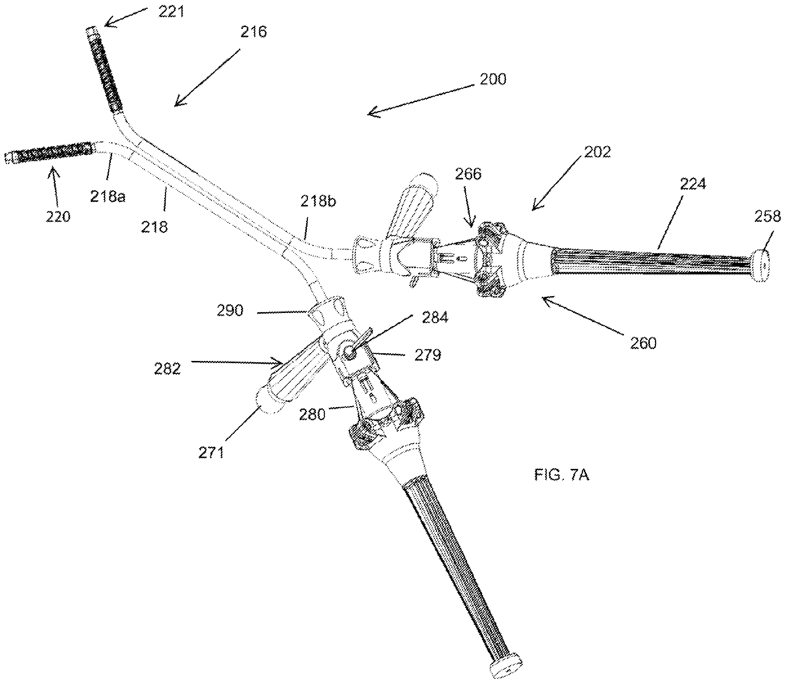

Fig. 7A shows an alternative embodiment of a deflectable port not according to the invention, which differs from the first embodiment in its use of a ball and socket type actuator to engage the pullwires to steer the flexible distal section of the instrument delivery tube. As with the first embodiment, this second embodiment is configured as an active, flexible-ended, port 200 which may function on its own as a laparoscopic surgical port. For example, three such active flexible ports 200 may be positioned in a manner similar to the way in which laparoscopic trocars are positioned for multi-port laparoscopic procedures. Alternatively, two or more such ports 200 may be employed through multi-instrument access devices, including the types described in U.S. Application Nos. 12/209,408 [US 2009/0227843], filed September 12, 2008, and 12/511,043 [US 2011/0060183], filed July 28, 2009. Fig. 7A shows two ports 200 as they might be positioned relative to one another when used through such a multi-instrument access device.

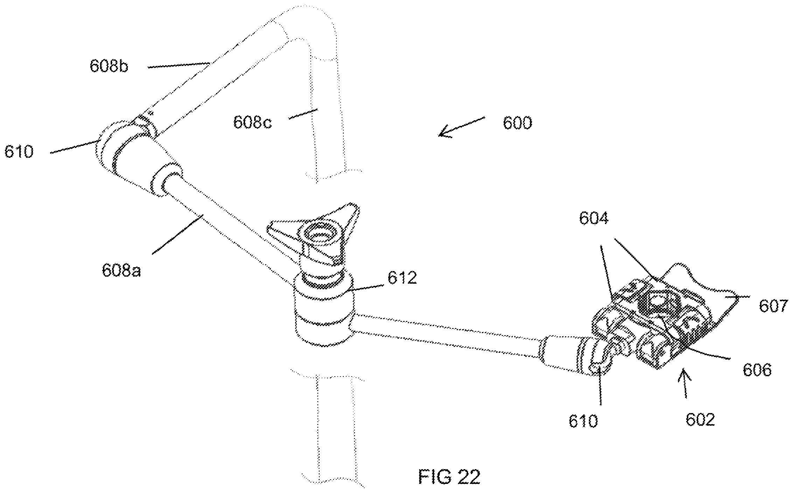

Referring to Fig. 7A, the port 200 includes an instrument delivery tube 216 which includes a rigid section 218 and a flexible section 220 distal to the rigid section 218. An actuator 202 on the proximal portion of the port 200 controls deflection of the flexible distal section 220 by engaging pull wires 280, allowing manipulation of the operative end of an instrument disposed within the instrument delivery tube 216. A device housing 279 supports the instrument delivery tube 216 and the actuator 202. The device housing 279 may include a handle 282 and/or a mount 271 for coupling the device to a support/stabilization arm coupled to an operating table, cart, operating room ceiling, or other operating room fixture. One example of a stabilization arm suitable for this purpose is described below with reference to Fig. 22. A mount for coupling to a stabilization arm may likewise be incorporated into the Fig. 1 port 10.

As with the first embodiment, the distal end of an instrument to be deployed into the body cavity via the port 200 is inserted into a control tube 224 on the actuator 202 and is then advanced into and through the instrument delivery tube. Manipulating the proximal handle of the instrument in turn moves the control tube 224, causing corresponding deflection of the distal end of the instrument.

Features of the instrument delivery tube of the port 200 will next be described with continued reference to Fig. 7A. The rigid section 218 comprises a rigid tube, which may be formed of stainless steel or other rigid tubing, having a fixed, preformed shape. In the Fig. 7A embodiment, the rigid tube 218 includes a generally straight main section, a distal region which includes a bend to create a curved or angled section 218a, and a curved or angled proximal section 218b. The curvature of the bend in the curved or angled section may be continuous or compound. The longitudinal axes of the straight and curved sections of the rigid tube 218 lie within a single plane, whereas in other embodiments different configurations may be used.

When two ports 200 are used adjacent to one another and positioned such that their distal sections 218a diverge as shown in Fig. 7A, the curve or angle of the distal section 218a separates the distal regions of the ports 200 while allowing the straight sections (which extend through the incision into the body) to be positioned side by side. The curve or angle of the proximal section 218b helps to separate the actuators so as to minimize conflict between them, and to also minimize conflict between handles of instruments positioned through the ports 200.

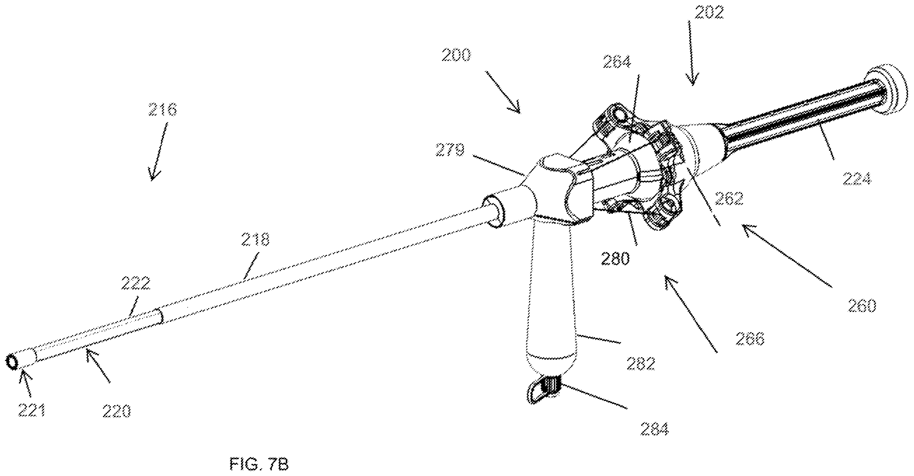

In the variation shown in Fig. 7B, the shaft of the rigid tube 218 is generally straight. In other embodiments, this shaft may have other shapes, including curved designs described in U.S. Application No. 12/209,408 [US 2009/0227843], filed September 12, 2008, entitled MULTI-INSTRUMENT ACCESS DEVICES AND SYSTEMS, U.S. Application No. 12/511,043 [US 2011/0060183], filed July 28, 2009, entitled MULTI-INSTRUMENT ACCESS DEVICES AND SYSTEMS, and U.S. Application No. 12/649,307 [US 2011/0230723], filed December 29, 2009, entitled ACTIVE INSTRUMENT PORT SYSTEM FOR MINIMALLY-INVASIVE SURGICAL PROCEDURES.

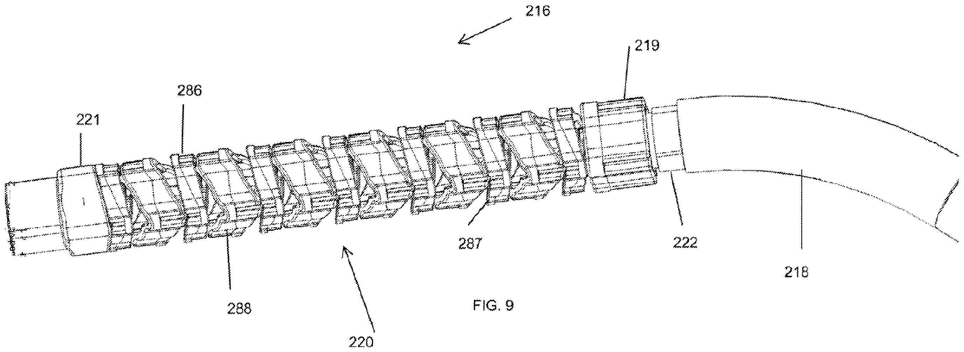

Referring to Fig. 9, the flexible distal section 220 in the Fig. 7A embodiment is constructed using a plurality of segments 286, 288 strung over the pullwires 280 (not shown in Fig. 9), which are anchored at or near the distal tip 221 of the instrument delivery tube 216. The segments 286, 288 and the distal tip include central bores that are longitudinally aligned to form a lumen. Segments 286, 288 are constructed to form rocker joints, such that adjacent segments can rock relative to one another in response to application of tension on the pull elements. Each segment 286, 288 includes guides 287 for receiving the pullwires. A lubricious liner extends through the central lumen defined by the segments 286, 288 to provide a smooth channel for movement of instruments through the central lumen. The segmented distal section 220 may be similar to the segmented sections found on the devices shown and described in U.S. Application No. 12/846,804 [US 2011/0251599], filed July 29, 2010, entitled DEFLECTABLE INSTRUMENT SHAFTS, Shellenberger et al, filed July 29, 2010 claiming priority to U.S. Provisional Application No. 61/323,863, filed April 13, 2010.

A flexible inner tube 222 extends through the rigid tube 218. The inner tube 222 has a distal end that terminates at location proximal to the segments 286, 288, and a proximal end disposed within the device housing 279. The inner tube 222 includes a lumen for receiving an instrument that is to be used within the body. Pull wires, cables, ribbons, or other actuation elements 280 extend through lumens in the wall of the inner tube 222, exit those lumens, and feed into the guides 287 in the segments 286, 288. In the preferred embodiment, each instrument delivery tube has four such wires arranged at 90 degree intervals. Other embodiments can utilize different numbers of pullwires, such as three pullwires equally spaced around the inner tube 222.

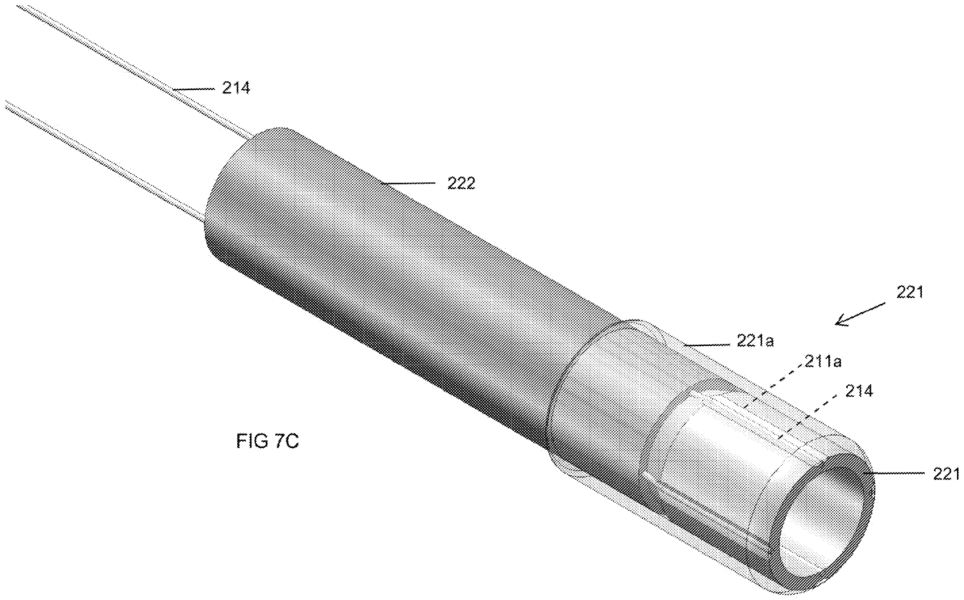

In the variation shown in Fig. 7B, the flexible distal section 220 is the exposed distal portion of flexible inner tube 222 that extends through the rigid tube 218. As with the Fig. 7A arrangement, the inner tube 222 includes a lumen for receiving an instrument that is to be used within the body and pullwire lumens (the distal ends of which are visible in Fig. 7C) for receiving the pullwires 280. The pullwires are anchored near the distal end of the inner tube 222 or within a tip section 221 coupled to the distal section.

Fig. 7C illustrates one configuration that may be used to anchor the pullwires, in which tip section 221 is an assembly that includes a tubular cap 221a and a tubular insert 211. Insert 211 has a plurality of longitudinal channels 211a longitudinally aligned with the pullwire lumens of the tube 222. When the device is assembled, insert 211 is held in alignment with the distal end of the tube 222 or physically coupled to the tube 222 such as by inserting its proximal end into the lumen of the tube 222. The pullwires 214 are laid in the channels 211a of the insert, and the tubular cap 221a is then press fit over the insert 211 and the distal end of the tube 222, capturing the pullwires 214 within the channels. This press fit technique for retraining the distal ends of the pullwires 214 may be used for each of the disclosed embodiments. Other techniques, such as crimping the distal ends of the pullwires 214 such that they cannot be retracted into the pullwire lumens, can also be used.

Since the pullwires for the flexible tube 222 are coupled to actuator 202, which acts on the pull-wires to deflect the distal section 220, the flexible inner tube 222 is constructed to be sufficiently flexible to allow the required deflection for instrument manipulation, while preferably also being resistant to kinking. In one embodiment, the flexible tube 222 is a composite tube formed using a PFTE inner liner lining the lumen, a thermal plastic sheath (having the pull wire lumens formed through it) overlaying the liner, a reinforcing layer over the thermal plastic sheath, and a second thermal plastic sheath over the reinforcing layer. In an alternate embodiment, the second thermal plastic sheath is eliminated and the reinforcing layer serves as the outer layer of the sheath. In yet another embodiment, the reinforcing layer may comprise the most inner layer of the tube. Various other embodiments, including those provided without reinforcing layers, or those having additional layers of reinforcing material or other materials can also be used.

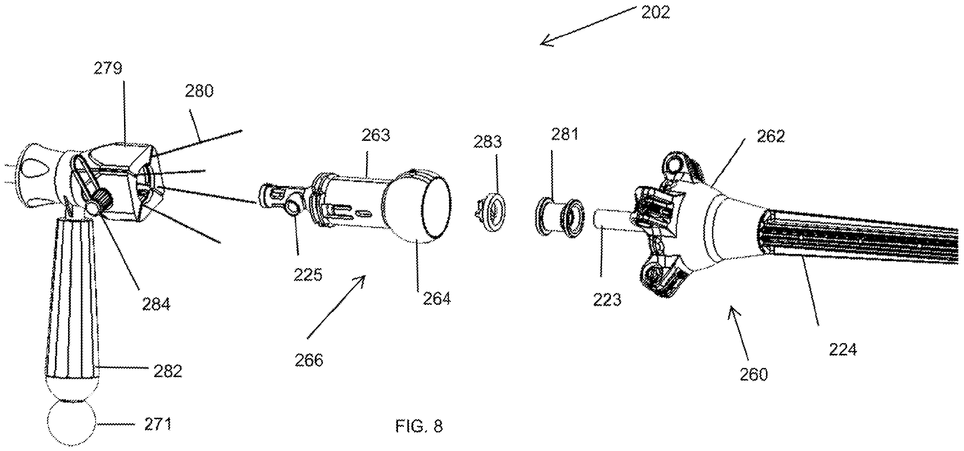

Fig. 8 shows details of the actuator 202, which may include features similar to those shown and described in U.S. Application Nos. 12/209,408 [US 2009/0227843], filed September 12, 2008, and 12/511,043 [US 2011/0060183], filed July 28, 2009. Each actuator 202 includes the control tube 224and a proximal entry port 258 for receiving a medical instrument. Entry port 258 includes a septum seal for sealing against the shaft of an instrument passed through it. The control tube 224 preferably has an inner tubular lining 223, preferably formed of a lubricious material such as PTFE or other suitable material so as to allow instruments inserted through the actuator to slide with ease. A proximal gimbal portion 260 is coupled to the distal end of the control tube 224. The proximal gimbal portion 260 has a distally-facing socket 262. A distal gimbal portion 266 includes a ball section 264 having a partially spherical surface partially disposed within the distally-facing socket 262 of the proximal gimbal section. The ball section further includes a tubular housing 270 that extends distally from the ball and into the device housing 279. The inner flexible tube 222 (not shown in Fig. 8, see Figs. 7A and 7B) extends into and is coupled to a reduced diameter distal part 263 of the tubular housing 270. A side opening 225 in the tubular housing 270 is positioned in the device housing 279 and is fluidly coupled to the luer port 284.

The tubular lining 223 extends through the proximal and distal gimbal portion 266 and has its distal end secured within the tubular housing 270 by a fitting 281. A valve 283, which may be a cross-slit duck bill valve, is disposed within the tubular housing 270. The valve functions to seal the actuator against loss of inflation pressure when no instruments are positioned through it.

The pullwires 280 exiting the proximal end of the flexible tube 220 extend out of the device housing 279 and are coupled to the proximal gimbal section 260.

During use of the actuation system, the shaft of an instrument I extends through the control tube 224, proximal gimbal portion, distal gimbal portion etc. and through the instrument delivery tube such that its operative end is disposed within the body cavity. A suitable instrument will have a rigid proximal section that will be disposed within or otherwise in contact with the control tube 224, and a flexible distal section. To articulate the distal end of the instrument, the surgeon moves the handle of that instrument, causing the control tube 224 to move with it. The proximal gimbal portion will move over the ball surface of the distal gimbal portion, thus tensioning the pullwires in accordance with the angle of the proximal gimbal portion relative to the distal gimbal portion. The distal portion of the instrument will deflect accordingly as a result of the action of the gimbal on the pullwires of the instrument delivery tube. Thus if it is desired to raise the distal end of the instrument, the user will lower the handle, moving the proximal gimbal section downwardly over the ball surface. This will thus apply tension to the upper pullwire 280, causing upward deflection of the instrument delivery tube as well as the distal end of the instrument. Lateral movement of the instrument shaft to the right will tension the corresponding side pullwire to cause the distal portion of the instrument delivery tube to bend to the left. The actuator system allows combinations of vertical and lateral deflection, giving 360° deflection to the instrument delivery tube. In other embodiments, the pullwires may be routed such that the movement of the flexible section 220 matches that of the control tube 224 (e.g. lifting the control tube lifts the distal end of the instrument delivery tube 216 and instrument).

The user may additionally advance/retract the tool longitudinally within the instrument delivery tube, and/or axially rotate the instrument within the instrument delivery tube when required. It should be noted that the positions of the ball and socket may be reversed, such that the proximal gimbal section includes a ball and the distal gimbal section has a socket within which the ball can articulate

If the port 200 is to function as a stand-alone port (i.e. rather than being introduced through a separate trocar or access device) the distal gimbal portion 266 may include or be coupled to a housing 280 shaped to seat within an incision (or other opening such as a trocar puncture) formed through a body wall (such as the abdominal wall). In the illustrated embodiment, the housing 280 is flared in a proximal direction to facilitate sealing within the incision. A handle 282 extends from the housing 280, allowing the user to manually support the port 200 (although the portion 200 may additionally or alternatively be provided with features such as mount 271 allowing its attachment to a support arm coupled to the surgical table.

A luer port 284 in the housing 279 (as in Fig. 7A), handle 282 (as in Fig. 7B), or another part of the housing may be fluidly coupled to the instrument delivery tube, allowing introduction of insufflation gas or irrigation fluid through the instrument delivery tube and into the body cavity.

The design of the illustrated embodiment allows the user to axially rotate the handle 282 relative to the longitudinal axis of the rigid tube 218, thereby allowing the user to select the orientation of the bends of the rigid tube 218 relative to the handle position. Thus, multiple units of the port 200 may be used for a single procedure, with each unit having its handle position selected to orient the bends of its corresponding rigid tube in a desired arrangement. For example, in Fig. 7A, two ports 200 are positioned with the port on the left having the bends of its rigid tubes oriented to be the reverse of the bends of the other one of the rigid tubes. This arrangement positions the distal and proximal ends of the ports such that they diverge from one another without requiring one of the handles 282 to be positioned upside down and without requiring different versions of the port to be manufactured (e.g. one having a left hand bend and one having a right hand bend).

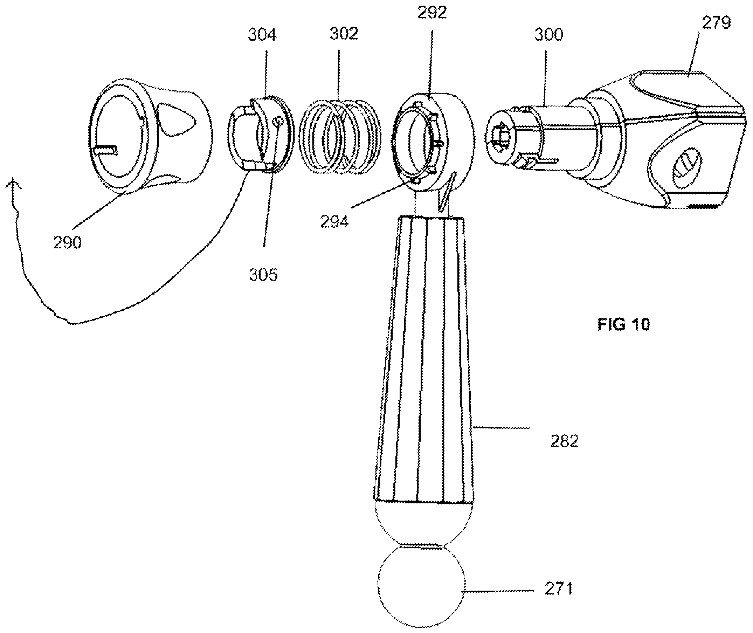

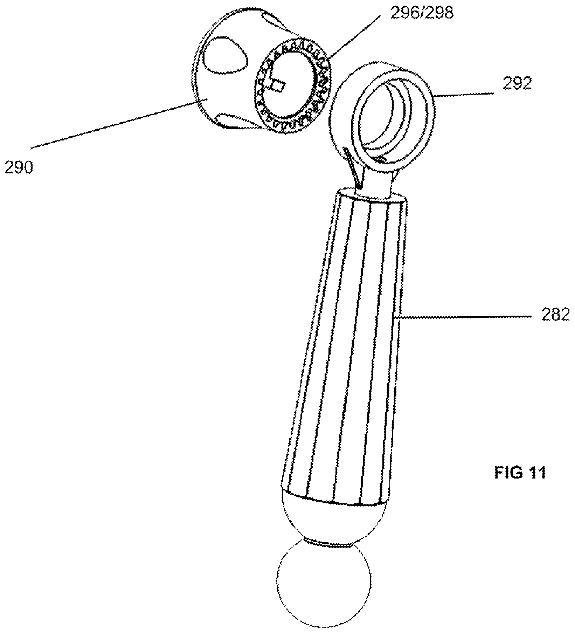

Fig. 10 shows one example of a mechanism permitting rotation of the handle 282 relative to the actuator 202 and instrument delivery tube 216. The handle 282 is coupled to a handle ring 292 having a plurality of radially positioned teeth 294 on its distal face. A coupler ring 290 has an inwardly-extending lip 296 forming a proximal face as shown in Fig. 11, and corresponding teeth 298 positioned on the lip 296.

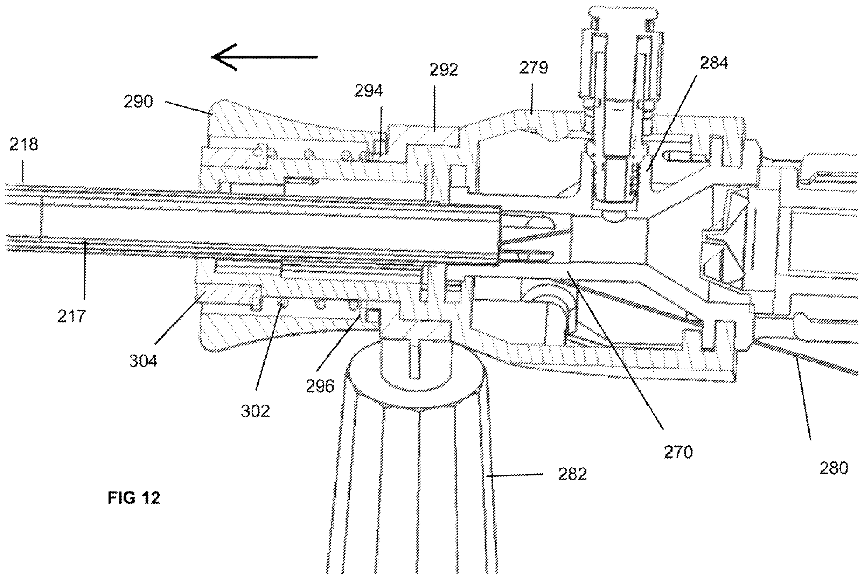

Referring again to Fig. 10, housing 279 includes a distal extension 300 that extends through the handle ring 292 and into the coupler ring 292. A compression spring 302 surrounds the distal extension 300. As best shown in Fig. 12, the compression spring 302 is retained by a sleeve 304 that is positioned around the distal extension 300 and coupled by pins 305 to the coupler ring 290. The compression spring 302 sits with its proximal end in contact with the distal surface of the lip 296 and with its distal end engaged by the sleeve 304. The spring 302 biases the coupler ring 290 in a proximal direction, such that its teeth 298 are engaged with the teeth 294 of the handle ring 292. To change the rotational position of the handle 282, the coupler ring 290 is pushed in a distal direction against the bias of the spring, as indicated by the arrow in Fig. 12, thereby disengaging the teeth 294, 298. The handle ring 292 is then free to axially rotate relative to the housing 279 by rotating the handle relative to the longitudinal axis of the instrument delivery tube 216. Once the handle is in a desired position, the coupling ring 290 is released by the user. The spring 302 moves the coupling ring 292 proximally such that the teeth 294, 298 re-engage, thus locking the handle against inadvertent axial repositioning.

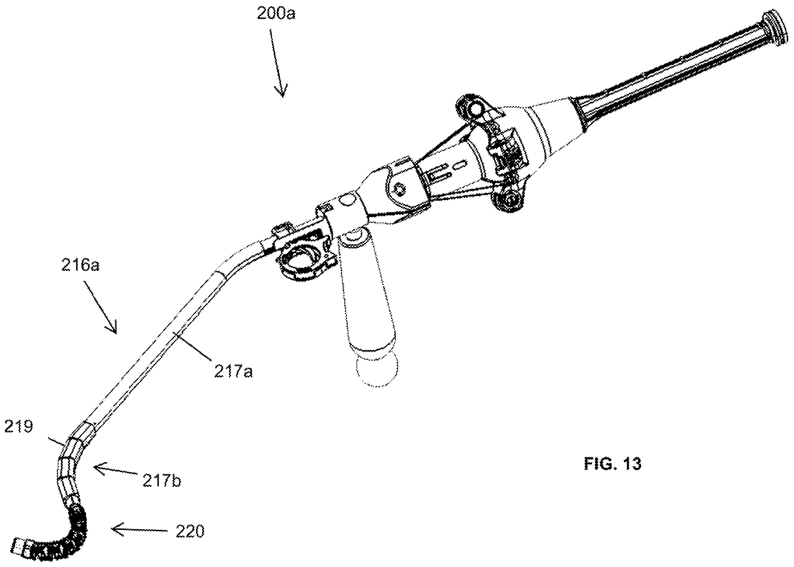

Fig. 13 shows an alternative port 200a not according to the invention, which is similar to the port 200 of Fig. 7A but which has been slightly modified to allow the distal end of the rigid tube (proximal to the flexible section 220) to have a state that is initially flexible to aid insertion of the port 200a through an access device or directly through an incision, but that may be subsequently made to assume a predetermined rigid shape. In this embodiment, rigid tube 218a includes a main rigid shaft 217a of fixed geometry, and a segmented shaft 217b formed of a plurality of shaft elements 219.

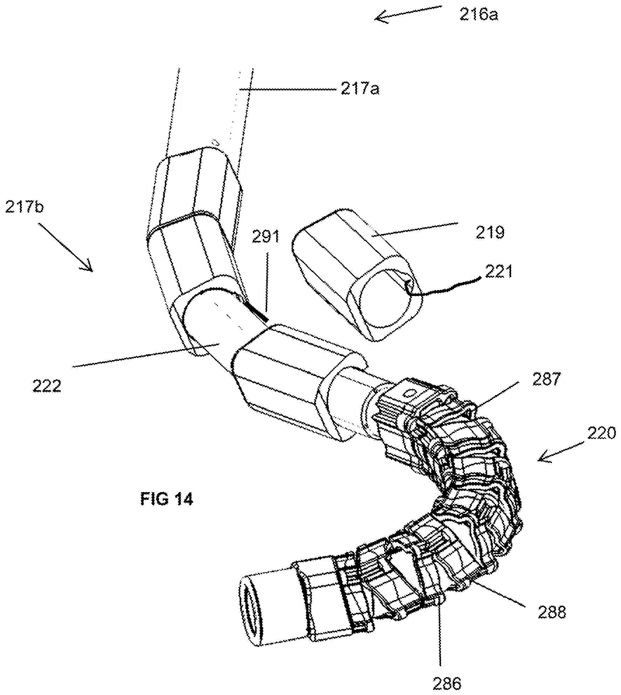

Fig. 14 shows the distal end of the instrument delivery tube 216a with one shaft element 219 separated from the remainder of the shaft. The shaft elements 219 are strung over the flexible tube 222. As with the prior embodiment, the flexible tube 222 includes pull wire lumens it is sidewalls, and the pull wires (not shown) exiting the distal ends of the pull wire lumens feed into guides 287 in the segments 286, 288 of flexible distal section 220. In this embodiment, an additional pull wire 291 is provided for converting the rigidizable section 217b to its rigid state. Pull wire 291 passes through the lumens of the shaft elements 219, along the outer surface of the flexible tube 222, and is connected at its distal end to element 293. The lumens of the shaft elements 219 may include a side channel or gap 221 to accommodate the pull wire 291.

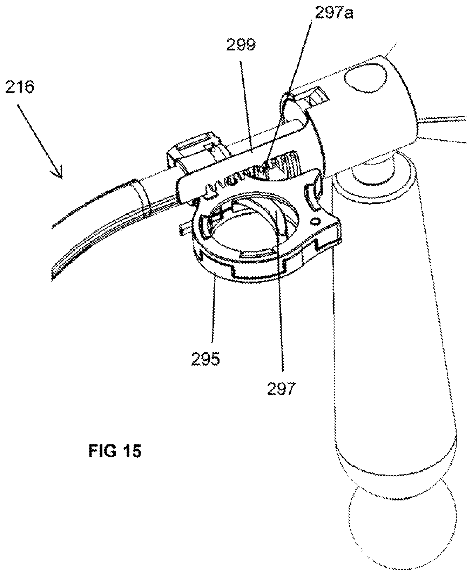

Referring to Fig. 15, the pullwire 291 is actuated using an actuation ring 295 coupled to the proximal end of the pullwire 291. The actuation ring is longitudinally slidable on the shaft of the instrument delivery tube 216a, such that withdrawing the actuation ring in a proximal direction converts the rigidizable section 217b to its rigid state. A locking system for retaining the section 217b in the rigid position comprises a trigger 297 carried by the actuation ring and having a wedge 297a pivotable into engagement with teeth of a ratchet sleeve 299. A leaf spring (not shown) biases the trigger 297 such that wedge is engaged with teeth of the ratchet sleeve 299 except when the trigger 297 is depressed by a user. To convert the rigidizable section 217b to its rigid state, the user depresses the trigger to unlock the locking system, then pulls the actuation ring proximally to tension the pullwire 291, and then releases the trigger 297 such that it reengages with the ratchet sleeve 299. To release the rigidizable section 217b to its flexible state, the locking system is unlocked by depressing the trigger, and then sliding the actuation ring 295 longitudinally forward to release the tension on the pullwire 291.

The shaft elements 219 are shaped such that when tension is applied to the pull wire 291, the distal face of each shaft element makes firm contact with the proximal face of its distally adjacent shaft element, and in doing so causes the shaft to assume a predetermined shape. The predetermined shape is preferably a curved shape, such as the one shown. It should be noted that the features of the axially rotatable handle described in connection with the Fig. 7A embodiment may be used in this embodiment, allowing the instrument delivery tube 216b to be positioned with the curvature of the shaft section 217b oriented in a desired direction.

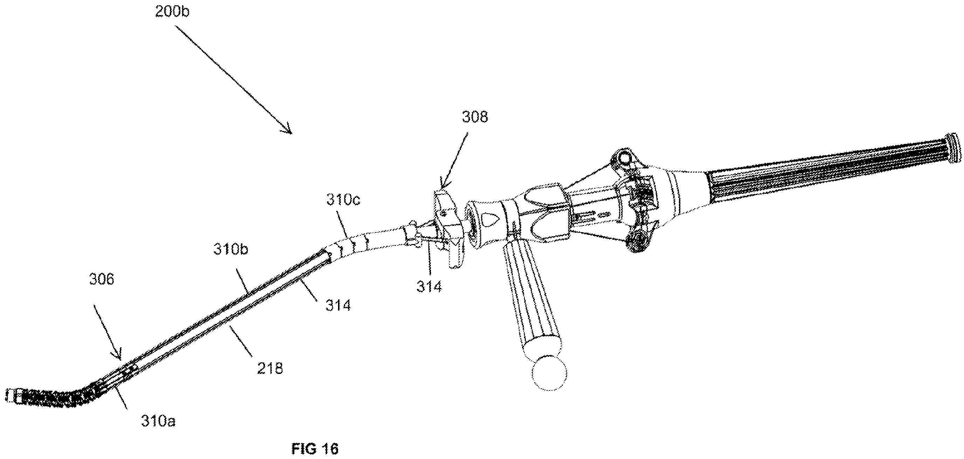

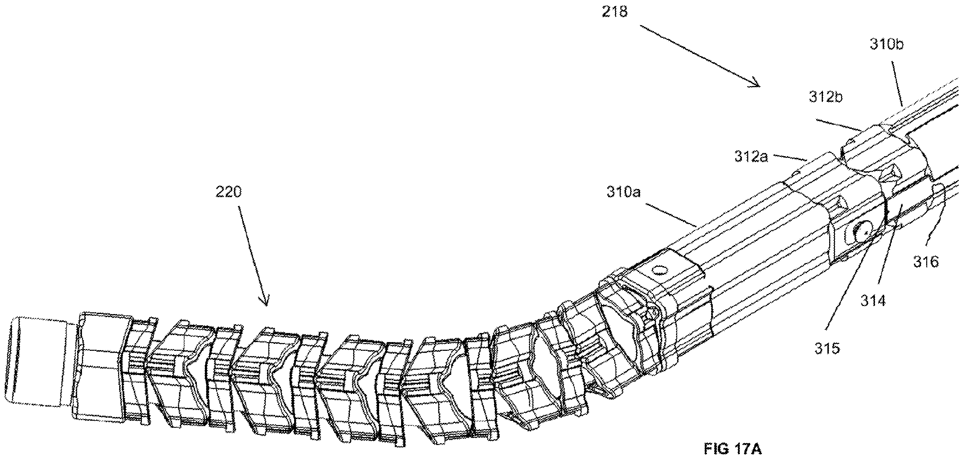

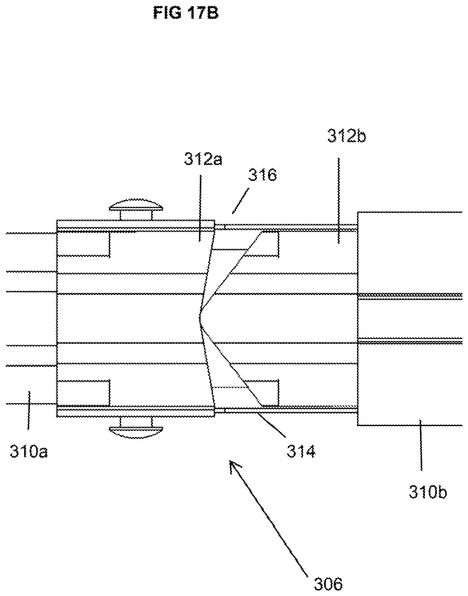

A port 200b according to the invention that is a variation of the Fig. 7A embodiment is shown in Fig. 16. The port 200b differs from the port 200 of Fig. 7A primarily in its inclusion of an articulation joint 306 at the distal end of the rigid tube 218 and an actuator for articulating the joint 306. The rigid tube 218 includes a distal member 310a proximally adjacent to the segments of the flexible section 220, an intermediate member 310b, and a proximal member 310c having a fixed curved shape. Referring to Fig. 17A, the articulation joint 306 is disposed between the distal and intermediate members 310a, 310b and comprises distal and proximal couplers 312a, 312b. The side elevation view of Fig. 17B best illustrates that the proximal face of the distal coupler 312a includes a convex surface or saddle, and the distal face of the proximal coupler 312b tapers to a peak which seats within that saddle, thereby forming a rocker joint.

A pair of elongate ribbons or sheets 314 of stainless steel or other suitable material have distal ends pivotally coupled to opposite sides of the distal coupler 312a. The sheets extend proximally along the outer surface of the proximal coupler 312b and through slots or recesses 316 formed in the outer surface of the intermediate member 310b. The sheets bend in regions 316 during articulation at the joint.

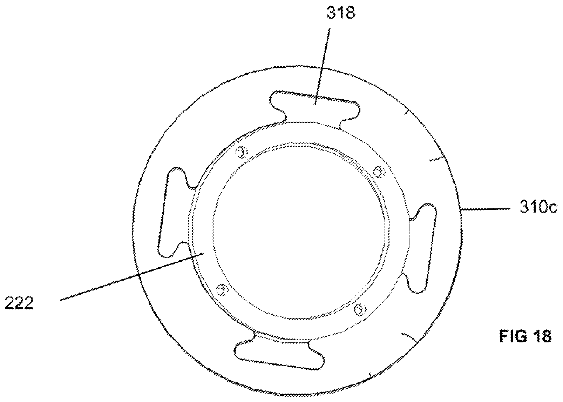

Referring again to Fig. 16, at the proximal member 310c of the rigid tube, the elongate sheets 314 pass into internal channels 318 (Fig. 18) disposed within the proximal member 310c. It should be noted that while the proximal member 310c of the rigid tube 218 is shown as being formed of a plurality of segments, a single piece might instead be used.

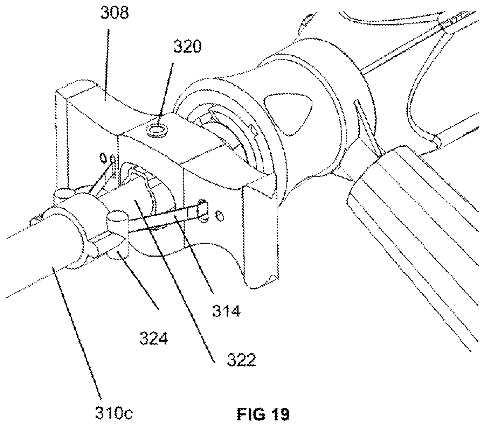

The proximal ends of the elongate sheets 314 exit the proximal end of the proximal member 310c and are secured to opposite side wings of the actuator 308 as shown in Fig. 19. The actuator 308 is mounted by a pivot, such as rivet 320, to a hypotube coupler 322 extending from the proximal end of the proximal member 310c. Pivoting the actuator 308 in one direction will withdraw one of the elongate sheets 314 while advancing the other of the elongate sheets, causing articulating of articulation joint 306 in one direction. Rollers 324 are positioned to allow each sheet 314 to curve around its corresponding roller when that side of the actuator is pivoted distally, thereby preventing the sheets 314 from kinking.

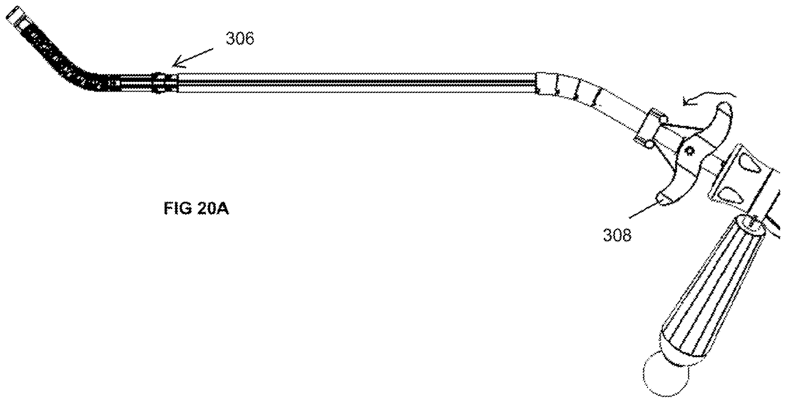

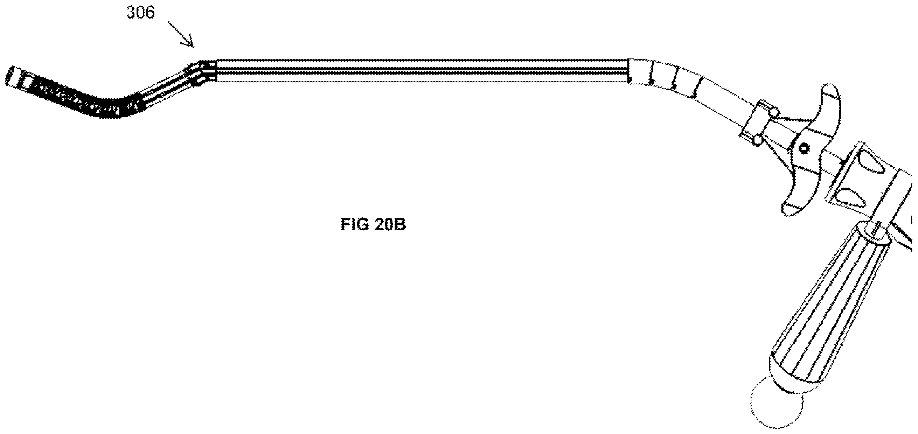

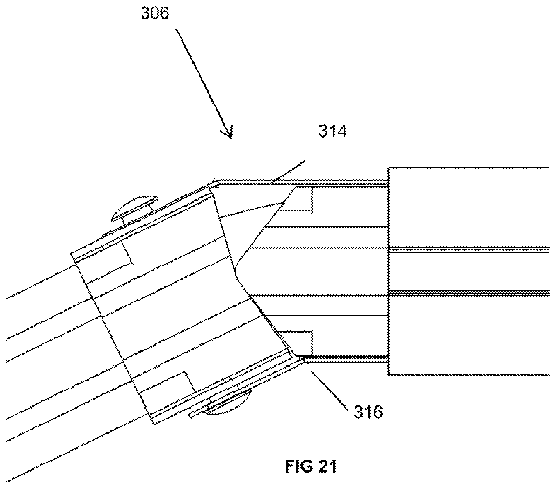

In use, the user manipulates the actuator to cause articulation of the articulation joint 306 in the desired direction. Pivoting the actuator 308 as shown by the arrow in Fig. 20A causes articulation of the articulation joint 306 into the position shown in Fig. 20B, whereas pivoting the actuator in the opposite direction will produce articulation in the opposite direction. Fig. 21 shows the position of the articulation joint 306 and the bending of the sheets 314 at bend regions 315 during articulation. The actuator may include a lock (not shown) for retaining the actuator in the pivoted position to retain the bend produced at the articulation section.

Fig. 22 shows an example of a stabilization arm 600 that may be used to support the disclosed ports. The stabilization arm may include features found in the stabilization arm sold by TransEnterix, Inc. of Durham, NC for use with the Spider™ Surgical System.

The stabilization arm 600 includes a clamp 602 designed to be coupled to the port's spherical mount 271 (Fig. 7A), allowing the port to be oriented in an unlimited number of positions. The clamp 602 includes clamp halves 604 that define an opening 606 for receiving the mount 271. A lever 607 is pivotable to draw the clamp halves towards one another to clamp the mount 271 between them. The clamp 602 is mounted to a collection of arm members 608a-c interconnected by universal (e.g. ball and socket) joints 610 or pivot elbow joints 612. The combination of joint allows the stabilization arm to support the port in any user-selected orientation. The proximal arm member 608c is coupled to the surgical table or to another fixture within the operating room.

To mount the port to the clamp 602, the spherical mount 271 or a port is disposed between the clamp halves. The user places the port in the desired three-dimensional orientation and then closes and latches the lever 606 to clamp the spherical mount 271. If at any time during the procedure the user wishes to adjust the orientation of the port, s/he may unlatch the clamp halves to do so. Given the universal nature of the coupling between the clamp and the spherical mount, and the presence of the adjustable joints 610, 612 between the arm members, the user may choose to alter the pitch, roll and/or yaw of the port.

The ports 10, 200, 200a, 200b described herein may be used in a variety of different types of procedures. Because the ports may be made as individual units that are not physically connected to one another, systems of ports may be used together but positioned and repositioned independently of one another. The following discuss describes a few examples of methods for using the ports, together with port systems (systems of components) that facilitate their use. In one example, two, three or more such active flexible ports may be positioned through separate incisions in a manner similar to the way in which laparoscopic trocars are positioned for multi-port laparoscopic procedures

This application allows surgery to be carried out in a manner that is similar to conventional laparoscopy, but allows for greater range of motion for the instruments than could be achieved using rigid instruments through conventional trocar ports. A port system for this application will include a plurality of access devices (if used), two or more ports 100, 200, 200a, 200b, and stabilization arms for the ports.

For this procedure, three or four incisions are formed through the skin and underlying tissue. A trocar or other sealed access device is positioned through each incision, and the distal end of each port is inserted into one of the access devices and advanced into the body cavity. Some of the access devices may be used to receive devices other than ports, such as scopes or staplers. If desired, the ports may be used without other access devices, in which case the distal ends of the ports are inserted directly through the incisions and advanced into the body cavity (although access devices may still be used for scopes or other instruments). Insufflation gas is directed through the port or the access device to inflate the body cavity. Each port is coupled to its own dedicated stabilization arm 600 (Fig. 22), placed in a desired orientation, and locked in the chosen orientation using the stabilization arm. Orienting the port may include adjusting the rotational position of the handle relative to the rigid tube as discussed in connection with Figs. 10 through 12. It should be noted that it may be preferably to orient the handle 282 of the port generally upwardly and to thus suspend the port from the stabilization arm 600.

If the Fig. 13 port 200a is used, the trigger 297 is engaged to draw the segments 219 into the curved, rigid orientation. If the Fig. 16 port 200b is used, the articulation joint 306 may be articulated to a give the distal end of the port a chosen orientation.

Flexible medical instruments to be used to perform the operative procedure are advanced through the ports, and their handles are manipulated to steer/deflect the distal ends of the ports through engagement of the actuators. If the Fig. 20A port embodiment is used, the articulation joint 306 may be articulated during the procedure to allow further adjustments to the positioning of the distal end of the medical instrument.

Gross positioning of the port within the incision may be adjusted during the procedure in a variety of ways. For example, pitch and yaw of the port may be adjusted at the stabilization arm. The port may be axially rolled within the incision by adjusting the rotational position of the rigid tube relative to the handle as discussed in connection with Figs. 10 through 12. Longitudinal advancement/retraction of the port relative to the incision allows "z-axis" movement of the port and corresponding instrument. Fine positioning of the instrument is likewise available, through deflection of the distal end of the port, axial rotation of the instrument within the port, or longitudinal or z-axis movement of the instrument within the port.

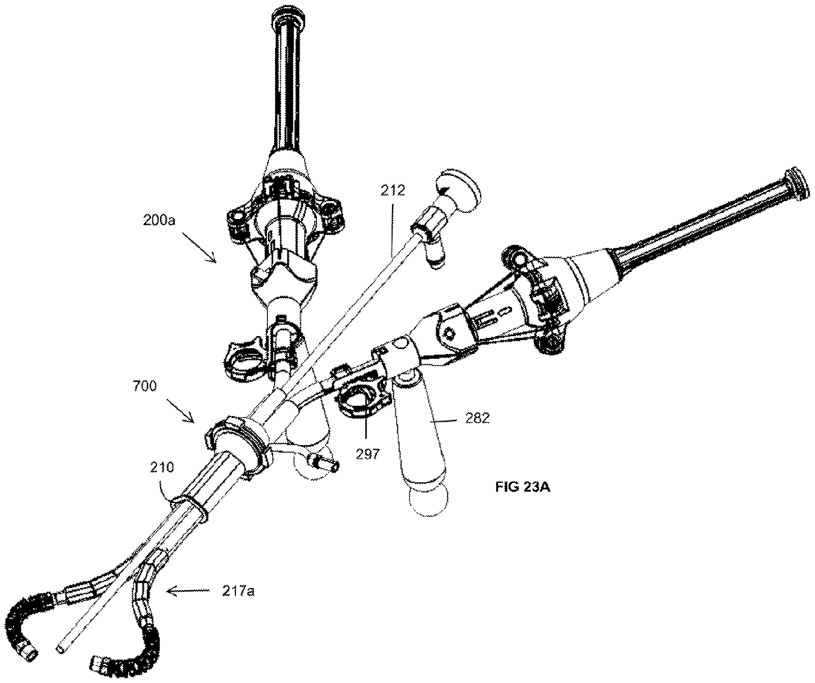

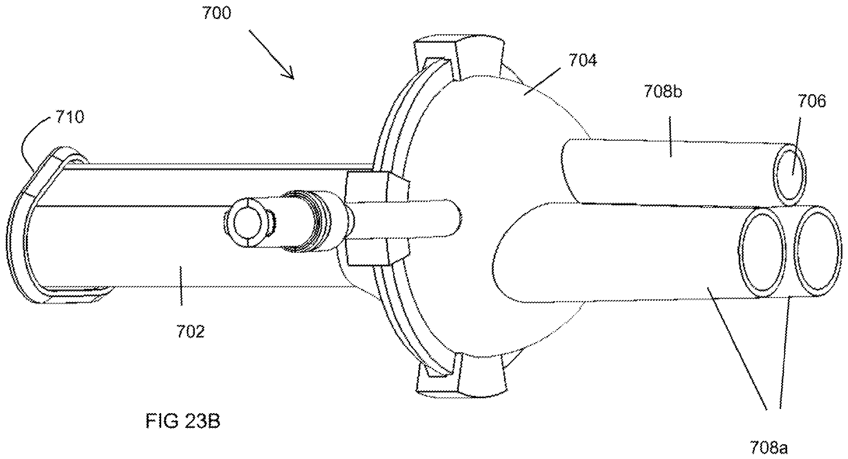

Fig. 23A illustrates a port system that includes two of the Fig. 13 ports in combination with a common access device 700 and stabilization arms (not shown) for one or both of the ports. Access device may have features similar to those described in U.S. Application No. 12/209,408 [US 2009/0227843], filed September 12, 2008, entitled MULTI-INSTRUMENT ACCESS DEVICES AND SYSTEMS. As shown in Fig. 23B, the access device 700 includes a base 702 positionable within an opening (e.g. an incision or puncture) formed in a body wall, and a seal 704 on the base. The seal is preferably positioned such that it is disposed outside the body wall during use. The seal may be removably attached to the base to allow large devices (e.g. gastric bands that are to be implanted using the system) to be passed directly through the base into the body cavity.

The seal 704 includes a plurality of openings 706 for receiving the ports and other instruments. Is this embodiment, the openings are found in tubular fingers 708a, 708b extending proximally from the base. The openings may be formed with equal diameters, or they may have different diameters. The Fig. 23B access device includes three such fingers, two side-by-side fingers 708a, and a third centered between and above the fingers 708a. The base 702 may have a generally triangular opening 710 to accommodate the shafts of ports/instruments used through this arrangement of fingers 708a, 708b. Valves (not shown) such as cross-slit or duck bill valves may be disposed within each finger to seal that finger against loss of insufflation pressure during times when the finger is not occupied by a port or other instrument. However, the seals may be eliminated from openings that will remain occupied by ports throughout the time that insufflation is needed. Gasket seals may also be present in the fingers to seal against the shafts of the ports or other instruments passed through them.

In use of the Fig. 23A system, an incision is formed through the skin and underlying tissue and the access device is positioned with the base 702 extending through the incision. The distal end of each port 200a is inserted into one of the fingers 708a and advanced into the body cavity. A scope 712 or other device (e.g. an optional third port if visualization is to be carried using a separate incision or through one of the ports) may be inserted into the body cavity via finger 708b. The body cavity is inflated using insufflation gas directed through the inflation port of the access device or through the luer ports on one of the ports 200a. Each port is coupled to its own dedicated stabilization arm 600 (Fig. 22), placed in a desired orientation, and locked in the chosen orientation using the techniques described above.

The handles 282 of the ports 200a may be oriented as shown, or they may extend generally upwardly (opposite to the illustrated direction) or in another direction for coupling to the stabilization arm. It also bears mention that the rotational position of each handle 282 is selected so that the bends of sections 217a have the desired orientation. Thus, to achieve the mirror-image orientation shown in Fig. 23A, each one of the shafts 217 is inverted about its longitudinal axis relative to the other shaft.

The trigger 297 for each port 200a is engaged to draw the segments 219 into the curved, rigid orientation, thus allowing separation of the ports 200a within the body.

Flexible medical instruments to be used to perform the operative procedure are advanced through the ports, and their handles are manipulated to steer/deflect the distal ends of the ports through engagement of the actuators. Adjustments to the positioning of the port 200a and instruments may be made throughout the procedure as discussed above.

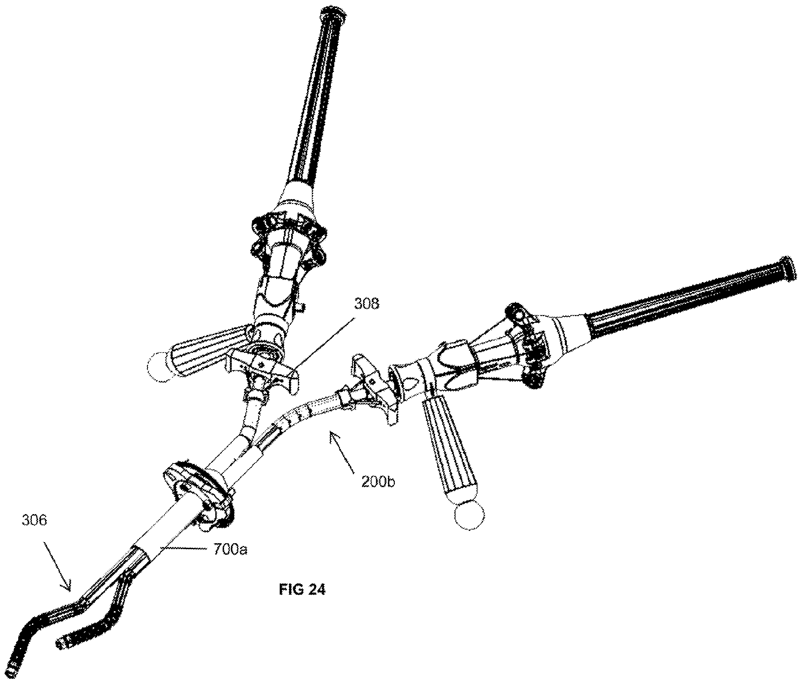

Fig. 24 shows use of a port system in which two of the ports 200b of Fig. 16 extend through a three finger access device 700a that is generally similar to the Fig. 23B access device 700. Use of this system is similar to use of the system described with respect to Fig. 23A, but includes use of the actuator 308 to manipulate the actuation joint 306, and locking of the actuator 308 to temporarily fix the angle of articulation. Fig. 24 shows the two ports 200b advanced different distances through the access device 700a, illustrating that use of the disclosed ports allows for independent z-axis positioning of the ports and their corresponding instruments.

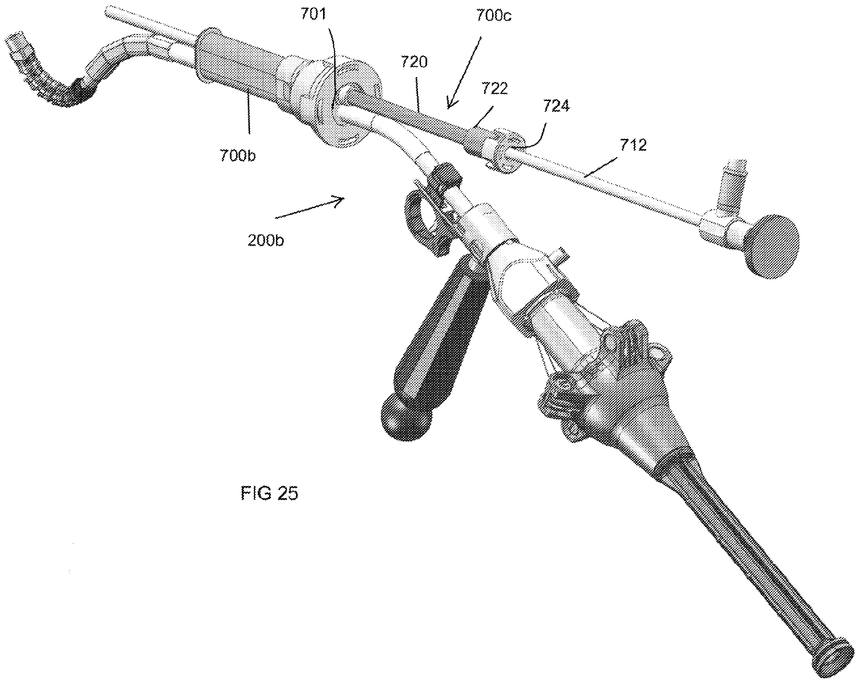

As another example, one of the disclosed ports 10, 200, 200a, 200b may be used to conduct single port biopsy procedures. A port system suitable for performing this procedure using the port 200a is shown in Fig. 25. In this type of procedure, the port 200a as well as an endoscope may be introduced through a trocar or other access device disposed within an incision in the body wall. Fig. 25 shows an access device 700b having an elastomeric seal 701 that includes a pair of openings for receiving shafts of instruments or ports (and preferably for sealing against those shafts). A duck bill or cross-slit valve may be provided within the access device 700b as discussed above. A port extension 700c is disposed in one of the openings. The port extension includes a rigid tube 720, a proximal housing 722 having a proximal opening 724, and preferably a seal that seals against instruments passed into the port extension. The seal may be a septum seal 726 that includes the opening 724 and that may be held on the housing 722 by a cap 728. Housing contains a valve or seal (e.g. a cross-slit seal 730 or duck bill valve) for sealing the port extension in the absence of instruments extending through it. The rigid tube 720 may optionally include a proximal connector 732, such as a flexible tubular plug insertable into an opening of the access device 700b as shown in Fig. 25. As with prior embodiments, the port system of Fig. 25 may include a stabilization arm (not shown).

Scope 712 is shown positioned through the port extension 700c. Although the port extension is optional, it gives the user an access point for scopes or instruments that is more proximal than the access point for the port 200b and thus that is lateral to the angled proximal portion of the port 200b. This allows the user to insert instruments through the access device 700b without his/her hand being constrained by the shaft of the port 200b.

Alternatively, the housing of the port 10, 200, 200a, 200b used for the biopsy procedure may include a lumen or a side car support for receiving an endoscope, allowing the port to be used without a separate trocar or access device. Similar arrangements may be used for transanal (TEM) procedures (e.g. polyp removal), transgastric procedures, transvaginal or transthoracic procedures. In some such procedures, two of the ports 10, 200, 200a, 200b may be disposed side by side through a natural orifice.

As another example, the port 10, 200, 200a, 200b may be passed down one of the passive ports of the access devices described in the prior applications described herein, for example the device disclosed in U.S. Application No. 12/649,307 [US 2011/0230723], filed December 29, 2009, entitled ACTIVE INSTRUMENT PORT SYSTEM FOR MINIMALLY-INVASIVE SURGICAL PROCEDURES, effectively adding an additional active port to those designs. In one application of this example for implantation of a gastric band for obesity therapy, the instrument delivery tubes of the active ports of those access devices may be used with grasping instruments operated to grasp tissue. The port 10, 200, 200a, 200b, which might extend through a passive port disposed between the active ports, could be used to manipulate a snare or other grasping device around the posterior side of the stomach in order to engage the gastric band and draw it around the stomach.

The listed examples of applications and port systems are merely representative and should not be considered comprehensive. Each of the disclosed ports and the port extender may be used with any of the disclosed access devices (as well as with others developed in the future or known to those skilled in the art, e.g. those described in US 2006/0020241, US 2008/0086167, US 2008/0255519 and elsewhere), and port systems may include multiple ports of the same type (e.g. as shown in Fig. 24) or combinations of ports of different types.

While certain embodiments have been described above, it should be understood that these embodiments are presented by way of example, and not limitation. It will be apparent to persons skilled in the relevant art that various changes in form and detail may be made therein without departing from the scope of the invention. This is especially true in light of technology and terms within the relevant art(s) that may be later developed.

Moreover, features of the various disclosed embodiments may be combined in various ways to produce various additional embodiments.

## Claims

1. An instrument port (200b) comprising:
an elongate tube having a lumen, the elongate tube comprising a rigid section (218) having a fixed shape, a deflectable section (220) distal to the rigid section and an articulating section (306, 310a) at the distal end of the rigid section and proximal to the deflectable section, the articulating section (306, 310a) pivotable from a generally straight position to an angled position, wherein the articulating section articulates independently of deflection of the deflectable section;
an actuator (202) coupled to the rigid section of the elongate tube, wherein the actuator includes an instrument pathway in communication with the lumen, the instrument pathway positioned such that a distal end of a medical instrument may be inserted through the instrument pathway and the lumen and out the distal end of the lumen into a body cavity;
a plurality of actuation elements (280) extending between the actuator (202) and the deflectable section, whereby manipulation of a proximal end of a medical instrument disposed in the instrument pathway and lumen engages the actuation elements to deflect the deflectable section;
a pair of elongate ribbons (314) extending proximally from the articulating section and an actuator (308) coupled to the proximal ends of the ribbons such that movement of the actuator articulates the articulating section;_and
a mount (271) coupled to the elongate tube, the mount engageable by an operating room stabilization arm.

2. The instrument port of claim 1, wherein the rigid section includes a fixed bend, and wherein the mount is rotatable from a first position to a second position relative to the longitudinal axis of the elongate tube to alter the orientation of the bend.

3. The instrument port of claim 2, wherein the mount is engageable in the first position and in the second position.

4. The instrument port of claim 1, further including a handle, wherein the mount is disposed on the handle.

5. The instrument port of claim 1, wherein the actuator (202) includes a proximal member and a distal member, wherein the actuation elements are coupled to the proximal member, and the proximal member being moveable relative to the distal member to engage the actuation elements and deflect the deflectable section.

6. The instrument port of claim 5, wherein a first one of the proximal and distal members is a ball, and the second one of the proximal and distal members is a socket, and wherein moving the proximal member relative to the distal member causes relative movement between the ball and socket.

7. The instrument port of claim 5, wherein a coil extends between the proximal and distal members, and wherein moving the proximal member relative to the distal member includes bends the coil.

8. The instrument port of claim 1, wherein the elongate tube includes a rigidizable section proximal to the deflectable section, the rigidizable section comprising a plurality of segments and a tensioning element extending through the segments, the tensioning element engageable to move the rigidizable section from a first, flexible, position, to a second, rigid, position having a predetermined curvature.

9. The instrument port of claim 1, wherein the rigid section includes a distal bend, a proximal bend, and a straight section between the distal and proximal bends.

10. The instrument port of claim 9, wherein the longitudinal axes of the distal and proximal bends and the straight section occupy a common plane.

11. The instrument port of claim 9, wherein the actuator (202) is positioned proximally of the proximal bend.

12. An instrument port system comprising:
an access device (700a) positionable in an incision through body tissue; and
an instrument port (200b) according to claim 1 independent of the access device, the instrument port insertable through and removable from the access device.

13. The instrument port system of claim 12, further including a second instrument port insertable through and removable from the access device independently of the first instrument port, the second instrument port comprising:
an elongate tube comprising a rigid section having a fixed shape and a deflectable section distal to the rigid section;
an actuator coupled to the rigid section of the elongate tube;
a plurality of actuation elements extending between the actuator and the deflectable section; and
a mount coupled to the elongate tube, the mount engageable by an operating room fixture.

14. The instrument port system of claim 12, wherein the access device includes first and second openings, wherein the instrument port is extendable through the first opening, and wherein the system further includes a port extender comprising an elongate rigid tube, a proximal housing, and a seal on the proximal housing, the port extender removably coupled to the second opening of the access device.

## Patentansprüche

1. Instrumentenport (200b), umfassend:
ein längliches Rohr mit einem Lumen, wobei das längliche Rohr Folgendes umfasst: einen steifen Abschnitt (218) mit einer festen Form, einen auslenkbaren Abschnitt (220) distal zu dem steifen Abschnitt und einen gelenkigen Abschnitt (306, 310a) am distalen Ende des steifen Abschnitts und proximal zum auslenkbaren Abschnitt, wobei der gelenkige Abschnitt (306, 310a) von einer im Allgemeinen geraden Position in eine abgewinkelte Position schwenkbar ist, wobei der gelenkige Abschnitt unabhängig von der Auslenkung des auslenkbaren Abschnitts gelenkig ist;
eine Bestätigungsvorrichtung (202), die mit dem steifen Abschnitt des länglichen Rohrs gekoppelt ist, wobei die Bestätigungsvorrichtung einen Instrumentenweg in Verbindung mit dem Lumen umfasst, wobei der Instrumentenweg derart positioniert ist, dass ein distales Ende eines medizinischen Instruments durch den Instrumentenweg und den Lumen und aus dem distalen Ende des Lumens in eine Körperhöhle eingeführt werden kann;
mehrere Betätigungselemente (280), die sich zwischen der Bestätigungsvorrichtung (202) und dem auslenkbaren Abschnitt erstrecken, wobei die Manipulation eines proximalen Endes eines in dem Instrumentenweg und dem Lumen angeordneten medizinischen Instruments mit den Betätigungselementen in Eingriff steht, um den auslenkbaren Abschnitt auszulenken;
ein Paar länglicher Bänder (314), die sich proximal von dem gelenkigen Abschnitt erstrecken, und eine Bestätigungsvorrichtung (308), die mit den proximalen Enden der Bänder derart verbunden ist, dass eine Bewegung der Bestätigungsvorrichtung den gelenkigen Abschnitt gelenkig bewegt; und
eine Halterung (271), die mit dem länglichen Rohr verbunden ist, wobei die Halterung mit einem Stabilisierungsarm des Operationsraums in Eingriff gebracht werden kann.

2. Instrumentenport nach Anspruch 1, wobei der steife Abschnitt eine feste Biegung umfasst, und wobei die Halterung von einer ersten Position in eine zweite Position relativ zur Längsachse des länglichen Rohrs drehbar ist, um die Ausrichtung der Biegung zu ändern.

3. Instrumentenport nach Anspruch 2, wobei die Halterung in der ersten Position und in der zweiten Position in Eingriff gebracht werden kann.

4. Instrumentenport nach Anspruch 1, ferner enthaltend einen Griff, wobei die Halterung auf dem Griff angeordnet ist.

5. Instrumentenport nach Anspruch 1, wobei die Betätigungsvorrichtung (202) ein proximales Element und ein distales Element umfasst, wobei die Betätigungselemente mit dem proximalen Element gekoppelt sind und das proximale Element relativ zu dem distalen Element beweglich ist, um mit den Betätigungselementen in Eingriff zu kommen und den auslenkbaren Abschnitt auszulenken.

6. Instrumentenport nach Anspruch 5, wobei ein erstes der proximalen und distalen Elemente eine Kugel ist, und das zweite der proximalen und distalen Elemente ein Sockel ist, und wobei das Bewegen des proximalen Elements relativ zu dem distalen Element eine relative Bewegung zwischen der Kugel und dem Sockel verursacht.

7. Instrumentenport nach Anspruch 5, wobei sich eine Spirale zwischen dem proximalen und dem distalen Element erstreckt, und wobei das Bewegen des proximalen Elements relativ zu dem distalen Element ein Biegen der Spirale beinhaltet.

8. Instrumentenport nach Anspruch 1, wobei das längliche Rohr einen versteifbaren Abschnitt proximal zu dem auslenkbaren Abschnitt beinhaltet, wobei der versteifbare Abschnitt mehrere Segmente und ein Spannelement umfasst, das sich durch die Segmente erstreckt, wobei das Spannelement in Eingriff gebracht werden kann, um den versteifbaren Abschnitt von einer ersten flexiblen Position in eine zweite steife Position mit einer vorbestimmten Krümmung zu bewegen.

9. Instrumentenport nach Anspruch 1, wobei der steife Abschnitt eine distale Biegung, eine proximale Biegung und einen geraden Abschnitt zwischen der distalen und der proximalen Biegung beinhaltet.

10. Instrumentenport nach Anspruch 9, wobei die Längsachsen der distalen und der proximalen Biegung und der gerade Abschnitt sich auf einer gemeinsamen Ebene befinden.

11. Instrumentenport nach Anspruch 9, wobei die Bestätigungsvorrichtung (202) der proximalen Biegung proximal angeordnet ist.

12. Instrumentenportsystem, umfassend:
eine Zugangsvorrichtung (700a), die in einem Einschnitt durch Körpergewebe positionierbar ist; und
einen Instrumentenport (200b) nach Anspruch 1, der von der Zugangsvorrichtung unabhängig ist, wobei der Instrumentenport durch die Zugangsvorrichtung einführbar und von dieser entfernbar ist.

13. Instrumentenportsystem nach Anspruch 12, ferner enthaltend einen zweiten Instrumentenport, welcher unabhängig von dem ersten Instrumentenport durch die Zugangsvorrichtung einführbar und von dieser entfernbar ist, wobei der zweite Instrumentenport Folgendes umfasst:
ein längliches Rohr, umfassend einen steifen Abschnitt mit einer festen Form und einen auslenkbaren Abschnitt distal zum steifen Abschnitt;
eine Betätigungsvorrichtung, die mit dem steifen Abschnitt des länglichen Rohrs gekoppelt ist;
mehrere Betätigungselemente, die sich zwischen dem Betätigungselement und dem auslenkbaren Abschnitt erstrecken; und
eine Halterung, die mit dem länglichen Rohr gekoppelt ist, wobei die Halterung durch eine Befestigungsvorrichtung des Operationsraums in Eingriff gebracht werden kann.

14. Instrumentenportsystem nach Anspruch 12, wobei die Zugangsvorrichtung erste und zweite Öffnungen umfasst, wobei der Instrumentenport durch die erste Öffnung verlängerbar ist, und wobei das System ferner Folgendes umfasst: einen Port-Extender, umfassend ein längliches steifes Rohr, ein proximales Gehäuse und eine Dichtung an dem proximalen Gehäuse, wobei der Port-Extender abnehmbar mit der zweiten Öffnung der Zugangsvorrichtung verbunden ist.

## Revendications

1. Port pour instrument (200b), comprenant :
un tube allongé comportant une lumière, le tube allongé comprenant une section rigide (218) de forme fixe, une section pouvant être déviée (220), distale à la section rigide, et une section d'articulation (306, 310a) au niveau de l'extrémité distale de la section rigide et à proximité de la section pouvant être déviée, la section d'articulation (306, 310a) pouvant pivoter d'une position généralement droite à une position inclinée, et la section d'articulation s'articulant indépendamment de la déviation de la section pouvant être déviée ;
un actionneur (202) accouplé à la section rigide du tube allongé, l'actionneur comprenant une voie d'instrument en communication avec la lumière, la voie d'instrument étant positionnée de sorte qu'une extrémité distale d'un instrument médical puisse être insérée à travers la voie d'instrument et la lumière et à l'extérieur de l'extrémité distale de la lumière dans une cavité corporelle ;
une pluralité d'éléments d'actionnement (280) s'étendant entre l'actionneur (202) et la section pouvant être déviée, la manipulation d'une extrémité proximale d'un instrument médical disposé dans la voie d'instrument et la lumière mettant en prise les éléments d'actionnement afin de dévier la section pouvant être déviée ;
une paire de rubans allongés (314) s'étendant de manière proximale à partir de la section d'articulation et un actionneur (308) accouplé aux extrémités proximales des rubans, de sorte que le mouvement de l'actionneur articule la section d'articulation ; et
un support (271) accouplé au tube allongé, le support pouvant venir en prise avec un bras de stabilisation de salle d'opération.

2. Port pour instrument selon la revendication 1, dans lequel la section rigide comprend une courbure fixe, et dans lequel le support peut tourner d'une première position à une seconde position par rapport à l'axe longitudinal du tube allongé pour modifier l'orientation de la courbure.

3. Port pour instrument selon la revendication 2, dans lequel le support peut venir en prise dans la première position et dans la seconde position.

4. Port pour instrument selon la revendication 1, comprenant en outre une poignée, le support étant disposé sur la poignée.

5. Port pour instrument selon la revendication 1, dans lequel l'actionneur (202) comprend un élément proximal et un élément distal, les éléments d'actionnement étant accouplés à l'élément proximal et l'élément proximal étant mobile par rapport à l'élément distal pour venir en prise avec les éléments d'actionnement et pour dévier la section pouvant être déviée.

6. Port pour instrument selon la revendication 5, dans lequel un premier élément des éléments proximal et distal est une bille et où le second des éléments proximal et distal est une cavité, le déplacement de l'élément proximal par rapport à l'élément distal entraînant un mouvement relatif entre la bille et la cavité.

7. Port pour instrument selon la revendication 5, dans lequel une bobine s'étend entre les éléments proximal et distal, le déplacement de l'élément proximal par rapport à l'élément distal comprenant des courbures de la bobine.

8. Port pour instrument selon la revendication 1, dans lequel le tube allongé comprend une section rigidifiable se trouvant à proximité de la section pouvant être déviée, la section rigidifiable comprenant une pluralité de segments et un élément de tension s'étendant à travers les segments, l'élément de tension pouvant venir en prise pour déplacer la section rigidifiable d'une première position flexible à une seconde position rigide de courbure prédéfinie.

9. Port pour instrument selon la revendication 1, dans lequel la section rigide comprend une courbure distale, une courbure proximale et une section droite, entre les courbures distale et proximale.

10. Port pour instrument selon la revendication 9, dans lequel les axes longitudinaux des courbures distale et proximale et la section droite occupent un plan commun.

11. Port pour instrument selon la revendication 9, dans lequel l'actionneur (202) est positionné à proximité de la courbure proximale.

12. Système de port pour instrument, comprenant :
un dispositif d'accès (700a) pouvant être positionné dans une incision à travers un tissu corporel ; et
un port pour instrument (200b) selon la revendication 1, indépendant du dispositif d'accès, le port pour instrument pouvant être inséré à travers le dispositif d'accès et retiré de celui-ci.

13. Système de port pour instrument selon la revendication 12, comprenant en outre un second port pour instrument pouvant être inséré à travers le dispositif d'accès et retiré de celui-ci indépendamment du premier port pour instrument, le second port pour instrument comprenant :
un tube allongé comprenant une section rigide ayant une forme fixe et une section pouvant être déviée distale par rapport à la section rigide ;
un actionneur accouplé à la section rigide du tube allongé ;
une pluralité d'éléments d'actionnement s'étendant entre l'actionneur et la section pouvant être déviée ; et
un support accouplé au tube allongé, le support pouvant venir en prise avec une fixation de salle d'opération.

14. Système de port pour instrument selon la revendication 12, dans lequel le dispositif d'accès comprend des première et seconde ouvertures, le port pour instrument pouvant s'étendre à travers la première ouverture et le système comprenant en outre une extension de port comprenant un tube rigide allongé, un boîtier proximal et un joint d'étanchéité sur le boîtier proximal, l'extension de port étant accouplée de manière amovible à la seconde ouverture du dispositif d'accès.
